# EUROPÄISCHE PATENTSCHRIFT

(11) **EP 4 615 342 B1**
(45) Veröffentlichungstag und Bekanntmachung des Hinweises auf die Patenterteilung: **18.11.2026**
(21) Anmeldenummer: 23798335.8
(22) Anmeldetag: 19.10.2023
(51) Int. Cl.: A61B 17/15, A61B 17/17, A61B 17/80

(54) **SCHABLONE FÜR EINE PELVIC-OSTEOTOMIE**
TEMPLATE FOR A PELVIC OSTEOTOMY
GABARIT POUR OSTÉOTOMIE PELVIENNE

(30) Priorität: 09.11.2022 AT 508522022
(43) Veröffentlichungstag der Anmeldung: 17.09.2025
(73) Patentinhaber: Tack GmbH, 2486 Pottendorf/Landegg (AT)
(72) Erfinder: TACK, Werner, 2486 Pottendorf-Landegg (AT); STEINMANN, Norbert, 72762 Reutlingen (DE)
(74) Vertreter: SONN Patentanwälte GmbH & Co KG
(86) Internationale Anmeldenummer: PCT/AT2023/060362
(87) Internationale Veröffentlichungsnummer: WO 2024/098084

(56) Entgegenhaltungen:
- EP-B1- 2 838 458
- WO-A1-2013/165558
- WO-A1-2022/143348
- US-A1- 2008 039 851

## Beschreibung

Die Erfindung betrifft eine Schablone zur Markierung einer Schnittlinie und zum Setzen von zumindest einer Bohrung am Darmbein für die Implantation einer Osteosynthese-Platte bei einer Pelvic-Osteotomie und ein Kit für eine Pelvic-Osteotomie.

Hüftdysplasie ist insbesondere bei Hunden eine häufige entwicklungsbedingte Skeletterkrankung, die zu einer Inkongruenz der Hüftgelenksoberfläche mit unzureichender Abdeckung des Hüftkopfes durch die dysplastische Hüftpfanne und einer sekundären Subluxation des Hüftkopfes führt. Zur chirurgischen Behandlung von Hüftdysplasie beispielsweise bei heranwachsenden Hunden, kann unter anderem eine Pelvic-Osteotomie im Rahmen einer "Triple Pelvic Osteotomy"(TPO), "Double Pelvic Osteotomy" (DPO) oder "Mono Pelvic Osteotomy" (MPO) durchgeführt werden. Dabei wird ein Schnitt durch das Darmbein gesetzt. Folglich kann der Teil des Beckens, der die Hüftpfanne enthält, leicht gedreht werden, um die Oberfläche der Hüftpfanne zu vergrößern, die den oberen Teil des Hüftkopfes (die "Kugel" des Hüftgelenks) bedeckt. Die zwei durch den Schnitt getrennten Knochenfragmente werden letztlich durch eine speziell geformte Knochenplatte miteinander verbunden. Aufgrund des Versatzes der beiden Knochenfragmente, weist die Knochenplatte zwei planare Abschnitte auf, die an jeweils einem Knochenfragment fixiert werden, die ebenso wie die Knochenfragmente zueinander versetzt sind und mittels eines Verbindungsabschnitts verbunden sind.

Eine "Double Pelvic Osteotomy" (DPO) und eine entsprechende Knochenplatte ist beispielsweise gezeigt in Zanetti, Elisabetta M., et al. "A structural numerical model for the optimization of double pelvic osteotomy in the early treatment of canine hip dysplasia." Veterinary and Comparative Orthopaedics and Traumatology oberseit.04 (2017): 256-264.

Auch in Vezzoni, A., et al. "Double pelvic osteotomy for the treatment of hip dysplasia in young dogs." Veterinary and Comparative Orthopaedics and Traumatology 23.06 (2010): 444-452 wird eine DPO und eine entsprechende Knochenplatte gezeigt.

Eine weitere Knochenplatte mit zwei Abschnitten und einer Übergangszone ist aus der EP 0 253 796 A2 bekannt.

Die CN 111 759 398 A zeigt eine Osteotomie-Lehre für eine Osteotomie am Darmbein, bei der das Darmbein in drei Fragmente zerteilt wird. Die Lehre hilft bei der Festlegung von Schnittlinien, um ein V-förmiges Fragment zu erhalten. Die Lehre umfasst ein Lineal, einen 180° Winkelmesser, einen 90° Winkelmesser und einen Messdraht.

Aus US 2008/0039851 A1 ist eine Osteotomieplatte mit einer Osteotomie-Schablone bekannt. Die Schablone umfasst ein längliches Element mit einem ersten Ende und einem zweiten Ende, ein erstes Schraubenloch, das in der Nähe des ersten Endes ausgebildet ist, und ein zweites Schraubenloch, das in der Nähe des zweiten Endes ausgebildet ist, wobei das erste und das zweite Schraubenloch so positioniert und dimensioniert sind, dass sie die mit der Osteotomieplatte verwendeten Knochenschrauben aufnehmen können. Die Schablone weist außerdem einen Spalt auf, der in der Nähe der Mitte der Schablone ausgebildet ist, wobei der Spalt so dimensioniert ist, dass er die Dicke eines Sägeblatts aufnehmen kann, das zur Durchführung der Osteotomie verwendet wird.

Aus EP 2 838 458 B1 ist ein orthopädisches Knochenfixiersystem und Verfahren zum Entwerfen eines Modells einer Knochenplatte bekannt.

WO 2022/143348 A1 offenbart eine Acetabulum-Seitenführungsplattenanordnung für den Ersatz eines Hüftgelenks und ein Verfahren zu deren Montage.

Aus WO 2013/165558 A1 ist ein Verfahren zur Herstellung einer patientenspezifischen chirurgischen Führung bekannt. Die Führung ist dazu eingerichtet, eine Bewegung eines Schneidwerkzeugs in Richtung eines Gewebekörpers zu führen.

Unter anderem aufgrund der speziellen Form der Knochenplatte mit zwei versetzten bzw. verdrehten Abschnitten ist deren Implantation mitunter schwierig umzusetzen. Einerseits ist die exakte Positionierung und Ausrichtung des Schnitts durch das Darmbein entscheidend für die Behandlung der (beginnenden) Hüftdysplasie.

Andererseits muss die Knochenplatte während der Operation an beiden Knochenfragmenten befestigt werden. Dazu sind in der Knochenplatte Durchgangsbohrungen vorgesehen, durch die die Knochenplatte beispielsweise mit Schrauben an den Knochenfragmenten fixiert werden kann. Um die Knochenplatte mittels Schrauben fixieren zu können, müssen entsprechende Bohrungen in die Knochenfragmente gesetzt werden. Dazu ist es notwendig, die Position der Bohrungen im Verhältnis zum Schnitt exakt festzulegen, um die Knochenplatte passend fixieren zu können. Eine freihändige korrekte Positionierung von Schnitt und Bohrungen ist nicht präzise reproduzierbar und hängt stark von der Erfahrung des Operateurs ab, der während der Operation nur eine eingeschränkte Sicht auf das Darmbein hat. Folgen von inkorrekt durchgeführten Operationen sind beispielsweise Nervenlähmung, Blutungen, Implantatversagen oder auch Knochenausrisse mit entsprechenden klinischen Folgen.

Es ist Aufgabe der Erfindung, Hilfsmittel bereitzustellen, welche die Durchführung einer Pelvic-Osteotomie erleichtern kann. Insbesondere ist es eine Aufgabe, eine Schablone bereitzustellen, mit der die Position und Ausrichtung einer Schnittlinie und zumindest einer Bohrung am Darmbein zur Implantation einer Osteosynthese-Knochenplatte einfach festlegbar ist.

Ein Verfahren zur Herstellung einer Schablone wird ebenfalls beschrieben, das keinen Teil der beanspruchten Erfindung bildet. Es dient lediglich dem Verständnis der Erfindung.

Das Verfahren weist zumindest die folgenden Schritte auf:
- Vorsehen der Osteosynthese-Platte mit zwei im Wesentlichen planen Plattenabschnitten, welche entlang einer Versetzungslinie zueinander versetzt sind, und mit mindestens einer ersten Durchgangsbohrung;
- Fertigen der an die Osteosynthese-Platte angepassten Schablone aufweisend eine Schablonenplatte mit einer Unterseite und einer Oberseite, wobei die Schablonenplatte ein Langloch aufweist, wobei das Langloch von der Oberseite zu der Unterseite der Schablonenplatte reicht und das Langloch das Markieren einer Schnittlinie am Darmbein durch das Langloch hindurch ermöglicht, und wobei die Schablonenplatte zumindest ein erstes Rundloch aufweist, wobei das zumindest eine erste Rundloch von der Oberseite der Schablonenplatte zu der Unterseite der Schablonenplatte reicht und durch das erste Rundloch eine Bohrung am Darmbein gesetzt werden kann, wobei die Position und Ausrichtung des Langlochs und des zumindest einen ersten Rundlochs durch eine Projektion der Osteosynthese-Platte auf die Schablonenplatte bestimmt wird, wobei die Position des ersten Rundlochs der Position eines durch die Projektion erzeugten Bildes der ersten Durchgangsbohrung auf der Schablonenplatte entspricht und wobei die Position und Ausrichtung des Langlochs der Position und Ausrichtung des Bildes der Versetzungslinie auf der Schablonenplatte entspricht.

Die Aufgabe wird von einer Schablone nach Anspruch 1 gelöst. Die Schablone ist vorzugsweise herstellbar, insbesondere hergestellt, nach dem Verfahren. Die Schablone weist zumindest eine Schablonenplatte mit einer Oberseite und einer Unterseite auf, wobei die Schablonenplatte ein Langloch aufweist, wobei das Langloch von der Oberseite zu der Unterseite der Schablonenplatte reicht und das Langloch das Markieren einer Schnittlinie am Darmbein durch das Langloch hindurch ermöglicht, und wobei die Schablonenplatte zumindest ein erstes Rundloch aufweist, wobei das zumindest eine erste Rundloch von der Oberseite der Schablonenplatte zu der Unterseite der Schablonenplatte reicht und durch das erste Rundloch eine Bohrung am Darmbein gesetzt werden kann.

Die Aufgabe wird auch von einem Kit für eine Pelvic-Osteotomie nach Anspruch 2 gelöst. Das Kit weist zumindest auf:
- eine Osteosynthese-Platte zur Implantation an einem Darmbein bei einer Pelvic-Osteotomie mit zwei im Wesentlichen planen Plattenabschnitten, welche entlang einer Versetzungslinie zueinander versetzt sind, und mit mindestens einer ersten Durchgangsbohrung zur Befestigung eines Knochenfragments, beispielsweise mittels zumindest einer Schraube;
- eine an die Osteosynthese-Platte angepasste Schablone, vorzugsweise herstellbar nach dem Verfahren, wobei die Schablone eine Schablonenplatte mit einer Oberseite und einer Unterseite aufweist, wobei die Schablonenplatte ein Langloch aufweist, wobei das Langloch von der Oberseite zu der Unterseite der Schablonenplatte reicht und das Langloch das Markieren einer Schnittlinie am Darmbein durch das Langloch hindurch ermöglicht, und wobei die Schablonenplatte zumindest ein erstes Rundloch aufweist, wobei das zumindest eine erste Rundloch von der Oberseite der Schablonenplatte zu der Unterseite der Schablonenplatte reicht und durch das erste Rundloch eine Bohrung am Darmbein gesetzt werden kann,
wobei die Position und Ausrichtung des Langlochs und des zumindest einen ersten Rundlochs durch eine Projektion der Osteosynthese-Platte auf die Schablonenplatte bestimmt ist, wobei die Position des ersten Rundlochs der Position eines durch die Projektion erzeugten Bildes der ersten Durchgangsbohrung auf der Schablonenplatte entspricht und wobei die Position und Ausrichtung des Langlochs der Position und Ausrichtung des Bildes der Versetzungslinie auf der Schablonenplatte entspricht.

Es ist eine Osteosynthese-Platte vorgesehen, die beispielsweise im Zuge einer Mono (MPO), Double (DPO) oder Triple Pelvic Osteotomy (TPO) am Darmbein implantiert werden kann. Die Osteosynthese-Platte weist zwei im Wesentlichen plane Plattenabschnitte auf, die jeweils an einem der in Folge eines Schnitts durch das Darmbein getrennten Knochenfragmente befestigt werden können. Die Plattenabschnitte der Osteosynthese-Platte sind mittels eines Verbindungsabschnitts miteinander verbunden. Der Verbindungsabschnitt verläuft typischerweise im Wesentlichen normal auf zumindest einen der Plattenabschnitte. Die an die Osteosynthese-Platte angepassten Schablone weist eine Schablonenplatte auf mit einer Unterseite und einer Oberseite, wobei im bestimmungsgemäßen Gebrauch die Unterseite der Schablonenplatte an das Darmbein angelegt ist. Folglich liegt eine Unterseite der beiden Plattenabschnitte und des Verbindungsabschnitts auf dem Darmbein bzw. dessen Knochenfragmenten auf. Die Unterseite des Verbindungsabschnitts liegt dabei im Wesentlichen an der Schnittfläche eines Knochenfragments des Darmbeins an. Das Knochenfragment, an dem die Unterseite des Verbindungsabschnitts anliegt ist das obere Knochenfragment bzw. das caudale Knochenfragment. Das Knochenfragment, an dem die Unterseite des Verbindungsabschnitts nicht anliegt ist das craniale untere Knochenfragment. Entsprechend dieser Definition weist auch die Osteosynthese-Platte einen oberen und einen unteren Plattenabschnitt auf. Der obere bzw. caudale Plattenabschnitt kann am caudalen oberen Knochenfragment fixiert werden, während der untere bzw. craniale Plattenabschnitt am cranialen unteren Knochenfragment fixiert werden kann. Die Osteosynthese-Platte weist zumindest eine erste Durchgangsbohrung in einer der beiden Plattenabschnitte auf, vorzugsweise am oberen Plattenabschnitt. Die Osteosynthese-Platte kann mehrere weitere Durchgangsbohrungen in jedem der Plattenabschnitte aufweisen, beispielsweise können pro Plattenabschnitt drei Durchgangsbohrungen vorgesehen sein. Die Osteosynthese-Platte kann die beiden Knochenfragmente in der vorgegebenen Position relativ zueinander fixieren. Die Osteosynthese-Platte bzw. deren Umriss kann beispielsweise so geformt sein, dass sie an den Iliumschaft anatomisch angepasst ist. Der Schnitt am Darmbein muss an der Stelle gesetzt werden, an der die Unterseite des Verbindungsabschnitts der Osteosynthese-Platte nach Implantation der Osteosynthese-Platte am Darmbein bzw. der Schnittfläche des oberen Knochenfragment aufliegen soll.

Die Schablone kann vor der Durchtrennung des Darmbeins, also vor Setzen eines Schnitts am Darmbein angelegt bzw. angebracht werden, um mittels des Langlochs Position und Ausrichtung des Schnitts zu markieren bzw. mittels des zumindest einen Rundlochs zumindest eine Bohrung zu setzen, sodass die Osteosynthese-Platte einfach implantiert werden kann. Das Langloch kann auch als Meißelschlitz bezeichnet werden. Die Schablonenplatte kann beispielsweise eine Höhe von 1 mm bis 10 mm aufweisen. Die Schablonenplatte kann beispielsweise eine Länge von 2 cm bis 10 cm und eine Breite von 0.7 cm bis 2 cm aufweisen. Die Schablonenplatte weist ein Langloch auf, das von der Oberseite zu der Unterseite der Schablonenplatte reicht.

Mittels des Langlochs bzw. durch das Langloch hindurch ist es möglich, eine Schnittlinie am Darmbein zu markieren. Das Langloch kann eine Meißelführung aufweisen, die gewährleistet, dass die Markierung normal auf die Schablonenplatte vorgenommen wird. Die Meißelführung kann beispielsweise durch eine Erhöhung der äußeren Begrenzung des Langlochs gebildet werden. Die Meißelführung kann beispielsweise im Wesentlichen ein elliptischer Hohlzylinder sein. Die Meißelführung kann durch die Schablonenplatte gebildet sein. Das Markieren der Schnittlinie kann durch Anritzen des Darmbeins mittels eines Meißels vorgenommen werden. Alternativ kann auch bereits zumindest ein Teil des Schnitts durch das Langloch bzw. die Meißelführung hindurch gesetzt werden.

Die Schablonenplatte weist zumindest ein erstes Rundloch auf, das von der Oberseite der Schablonenplatte zu der Unterseite der Schablonenplatte reicht. Durch das erste Rundloch kann eine Bohrung am Darmbein gesetzt werden. Das zumindest erste Rundloch weißt in einer Draufsicht einen runden insbesondere kreisförmigen Querschnitt auf und kann optional von der Oberseite der Schablonenplatte zur Unterseite der Schablonenplatte konisch zusammenlaufen.

Die Position und Ausrichtung des Langlochs sowie des ersten Rundlochs werden durch eine Projektion der Osteosynthese-Platte auf die Schablonenplatte bestimmt. Die Position des ersten Rundlochs entspricht der Position eines durch die Projektion erzeugten Bildes der ersten Durchgangsbohrung der Osteosynthese-Platte auf der Schablonenplatte. Die Position und Ausrichtung des Langlochs entspricht der Position und Ausrichtung des Bildes einer Versetzungslinie auf der Schablonenplatte. Das Langloch der Schablone ist in Relation zum Darmbein an der späteren Position der Unterseite des Verbindungsabschnitts. Die Versetzungslinie verläuft daher an der Unterseite der Osteosynthese-Platte entlang einer Innenkante, die am Übergang des oberen Plattenabschnitts mit dem Verbindungsabschnitt vorliegt. Die Innenkante ist eindeutig definiert, da die Unterseite der Osteosynthese Platte nur eine Innenkante am Übergang eines der Plattenabschnitte, insbesondere des oberen Plattenabschnitts mit dem Verbindungsabschnitt, aufweist. Durch die Projektion entspricht der Normalabstand der Längsachse des Langlochs zum Mittelpunkt des Rundlochs auf der Schablonenplatte dem Normalabstand der Innenkante zum Mittelpunkt der ersten Durchgangsbohrung auf der Osteosynthese-Platte.

Nach Entfernen der Schablone kann ein Schnitt entlang der markierten Schnittlinie durch das Darmbein gesetzt werden. Die zwei durch den Schnitt getrennte Knochenfragmente können versetzt werden, sodass die Osteosynthese-Platte, beispielsweise mittels Schrauben, an den beiden Knochenfragmenten befestigt werden kann. Die Schrauben können beispielsweise unidirektionale oder multidirektionale winkelstabile Schrauben oder Kortikalisschrauben sein. Die Osteosynthese-Platte kann beispielsweise zuerst mittels einer Schraube, die in das zumindest eine Bohrloch und die entsprechende erste Durchgangsbohrung am oberen Plattenabschnitt der Osteosynthese-Platte eingreift, am oberen Knochenfragment fixiert werden. Aufgrund des Anliegens der Unterseite des Versetzungsabschnitts an der Schnittfläche des oberen Knochenfragments, kann die Osteosynthese-Platte nicht um die Hauptachse der Schraube gedreht werden. Somit kann die Position der Osteosynthese-Platte in Relation zu dem oberen Knochenfragment definiert werden. Um die Osteosynthese-Platte auf dem unteren Knochenfragment zu fixieren, kann das zweite Knochenfragment am zweiten Plattenabschnitt der Osteosynthese-Platte angelegt werden. Es können Bohrungen in das untere und das obere Knochenfragment durch weitere Durchgangsbohrungen der Osteosynthese-Platte hindurch gesetzt werden, um letztlich beispielsweise mittels Schrauben die Osteosynthese-Platte an beiden Knochenfragmenten zu fixieren. Die Verwendung der Schablone kann daher die Implantation einer Osteosynthese-Knochenplatte deutlich vereinfachen.

Die Schablone weist an der Oberseite der Schablonenplatte einen abnehmbaren Positioniergriff auf, der zur Positionierung der Schablonenplatte am Darmbein verwendbar ist. Der Positioniergriff kann beispielsweise durch das erste Rundloch oder ein weiteres Rundloch der Schablonenplatte mittels eines Kirschner-Drahts am Darmbein befestigbar sein. Der Positioniergriff kann beispielsweise ein Stab sein, der im Vergleich zur Schablone insbesondere während einer Operation einfacher zu greifen ist. Der Positioniergriff kann an der dem Darmbein zugewandten Seite einen zylinderförmigen Abschnitt aufweisen, der im Wesentlichen formschlüssig in das erste Rundloch oder ein weiteres Rundloch eingebracht werden kann, um die Schablonenplatte lateral verschieben zu können. Dazu kann der Radius des zylinderförmigen Abschnitts im Wesentlichen dem Radius eines Rundlochs auf der Schablonenplatte entsprechen. Eine etwaige Befestigung des Positioniergriffs am Darmbein kann nach der korrekten Ausrichtung der Schablone erfolgen.

Der Positioniergriff weist einen Ausrichtstab auf, wobei die Hauptsachse des Ausrichtstabs normal auf die Hauptachse des Positioniergriffs steht. Die Verwendung eines Ausrichtstabs kann die korrekte Positionierung der Schablonenplatte und in Folge auch die Positionierung des Schnitts durch das Darmbeim und die Positionierung der Osteosynthese-Platte vereinfachen. Die Pelvic-Osteotomie kann normal zu einer imaginären Linie erfolgen, welche cranial vom Übergang des mittleren in das ventrale Drittel der Darmbeinschaufel und caudal auf Höhe des Sitzbeinhöckers verläuft. Der Ausrichtstab kann während einer Operation entlang dieser imaginären bzw. gedachten Linie ausgerichtet werden und so die Ausrichtung der Schablonenplatte vereinfachen.

Die Projektion kann beispielsweise eine Orthogonalprojektion sein, wobei für die Projektion die Haupterstreckungsebene eines der zwei im Wesentlichen planen Plattenabschnitten der Osteosynthese-Platte parallel zur Haupterstreckungsebene der Schabloneplatte ist. Vorzugsweise ist die erste Durchgangsbohrung im oberen Plattenabschnitt und die Haupterstreckungsebener des oberen Plattenabschnitts ist parallel zur Haupterstreckungsebener der Schablonenplatte für die Projektion. Durch die Orhogonalprojektion und die Ausrichtung der Haupterstreckungsebenen, wird das (geometrische) Verhältnis der Versetzungslinie zu der ersten Durchgangsbohrung unverzerrt auf die Schablonenplatte abgebildet. Die Position des Rundlochs auf der Schablonenplatte wird in Folge so bestimmt, dass sie der Position des Bildes der ersten Durchgangsbohrung entspricht. Die Position und Ausrichtung des Langlochs wird so bestimmt, dass sie der Position und Ausrichtung des Bildes der Versetzungslinie entspricht. Die Relation der Versetzungslinie zur ersten Durchgangsbohrung entspricht folglich der Relation des Bildes der Versetzungslinie auf der Schablonenplatte zum Bild der ersten Durchgangsbohrung auf der Schablonenplatte.

Ein Umriss der Schablonenplatte kann derart gewählt werden, dass der Umriss im Wesentlichen einer Außenkontur des durch die Projektion erzeugten Bildes der Osteosynthese-Platte auf der Schablonenplatte entspricht. Durch diese Wahl des Umrisses kann die die Unterseite der Schablonenplatte auf einen lateralen und im Wesentlichen planen Bereich des Darmbeins angelegt werden. Die Osteosynthese-Platte bzw. deren beide Plattenabschnitte müssen zur Implantation jeweils an ein Knochenfragment im Wesentlichen plan und/oder formschlüssig anlegbar sein. Wird der Umriss der Schablonenplatte entsprechend gewählt, kann somit auch die Schablonenplatte auf den entsprechenden Bereich des Darmbeins vor dem Setzten des Schnitts im Wesentlichen plan und/oder formschlüssig angelegt werden. Die Außenkante der Unterseite der Schablonenplatte kann abgeschrägt sein bzw. eine Fase aufweisen, um die Auflage am Knochen zu verbessern. Alternativ kann die Außenkante abgerundet sein.

Beispielswese kann die Schablonenplatte mittels einer additiven Methode, vorzugsweise 3D-Druck, oder mittels einer subtraktiven Methode, vorzugsweise Fräsen oder Bohren, gefertigt werden. Die Verwendung von 3D-Druck bietet eine besonders einfache und günstige Möglichkeit, die Schablonenplatte zu fertigen. Subtraktive Methoden können beispielsweise im Zusammenhang mit der Verwendung eines Metalls als Ausgangsmaterial für die Schablonenplatte verwendet werden.

Alternativ kann die Schablonenplatte mittels Spritzgusses gefertigt werden. Besonders mittels Spritzgusses ist die Fertigung einer hohen Anzahl an im Wesentlichen identen Schablonenplatten besonders kostengünstig möglich.

Die Schablonenplatte kann beispielsweise aus einem röntgendichten Material gefertigt werden. Die Schablonenplatte kann daher röntgendichtes Material aufweisen oder aus röntgendichtem Material bestehen, beispielsweise Stahl oder Titan. Röntgendicht bedeutet in diesem Fall, dass der Kontrast zwischen der röntgendichten Schablonenplatte und dem Darmbein genügend hoch ist, um eine Kontrolle der Position und/oder Ausrichtung der Schablonenplatte am Darmbein mit Hilfe von Röntgenstrahlung, beispielsweise mittels eines C-Bogens, zu ermöglichen. Vorzugsweise hat das röntgendichte Material eine CT-Zahl von mindestens 400 Hounsfield Units (HU), bevorzugt mindestens 1.000 HU, insbesondere mindestens 2.000 HU. Beispielsweise kann die Schablonenplatte Metall, beispielsweise Stahl, Titan, Aluminium oder eine Eisenlegierung, aufweisen.

Die Osteosynthese-Platte kann mindestens eine weitere Durchgangsbohrung zur Befestigung eines Knochenfragments und die Schablonenplatte zumindest ein weiteres Rundloch aufweisen, wobei das weitere Rundloch im Vergleich zum ersten Rundloch an einer gegenüberliegenden Seite der Schablonenplatte bezüglich des Langloches angeordnet ist, wobei die Position des weiteren Rundlochs der Position eines durch die Projektion erzeugten Bildes der weitere Durchgangsbohrung auf der Schablonenplatte entspricht. Die weitere Durchgangsbohrung kann auf dem unteren Plattenabschnitt sein. Die Position des weiteren Rundlochs kann durch eine zweite Projektion bestimmt werden, für die die Haupterstreckungsebene des unteren Plattenabschnitts parallel zur Haupterstreckungsebene der Schablonenplatte ist. Die zweite Projektion kann eine Orthogonalprojektion sein. Die Position und Ausrichtung des durch die zweite Projektion erzeugten Bildes der Versetzungslinie deckt sich mit der Position und Ausrichtung des Langlochs auf der Schablonenplatte. Die erste und die zweite Projektion führen im Wesentlichen zum selben Bild der Versetzungslinie auf der Schablonenplatte. Durch das Bild der weiteren Durchgangsbohrung kann die Position des weiteren Rundlochs definiert werden. Mittels des weiteren Rundlochs kann das untere Knochenfragment am unteren Plattenabschnitt fixiert werden. Die Osteosynthese-Platte kann mehrere Durchgangsbohrungen aufweisen und es können entsprechende mehrere Rundlöcher in der Schablonenplatte vorgesehen sein. Es können auch weniger Rundlöcher in der Schablonenplatte als Durchgangsbohrungen in der Osteosynthese-Platte vorgesehen sein, da auch durch Durchgangsbohrungen der Osteosynthese-Platte hindurch Bohrungen gesetzt werden können. Bohrungen, die mit Hilfe der Schablone gesetzt werden, können zu einer initialen Fixierung der Osteosynthese-Platte verwendet werden.

Beispielsweise kann an der Oberseite der Schablonenplatte zumindest eine Bohrführung angeordnet sein, wobei die Hauptachse der zumindest einen Bohrführung mit der Hauptachse des zumindest einen Rundlochs zusammenfällt. Durch die Bohrführung kann der Winkel der Bohrung im Darmbein bzw. der Eintrittswinkel der Bohrung besser definiert werden. Die Bohrung kann durch die Bohrführung und das Rundloch hindurch gesetzt werden. Die Bohrführung kann beispielsweise ein Hohlzylinder sein. Die Bohrführung ist besonders bei Verwendung von unidirektionalen Schrauben vorteilhaft.

Die Schablonenplatte kann zumindest eine Aussparung aufweisen, die eine Palpation des Darmbeins erlaubt, wenn die Schablonenplatte am Darmbein angelegt ist. Die Aussparung kann damit die Positionierung der Schablone vereinfachen. Es können auch weitere Aussparungen vorgesehen sein, die als Sichtfenster verwendet werden können.

Die Schablonenplatte kann zumindest zwei Löcher aufweisen, durch die jeweils ein Fixiermittel, vorzugsweise ein Kirschner-Draht, eine Fixierung der Schablonenplatte am Darmbein ermöglichen kann. Es kann vorteilhaft sein, die Schablone bzw. die Schablonenplatte vor dem Markieren der Schnittlinie und vor dem Setzen der Bohrung durch das Rundloch am Darmbein zu fixieren und so ein Verrutschen der Schablone zu vermeiden. Zwei dafür vorgesehene Löcher können die Fixierung der Schablonenplatte ermöglichen oder vereinfachen. Die Fixierung mittels Kirschner-Draht ist beispielsweise eine gängige, einfache und minimal-invasive Möglichkeit die Schablonenplatte zu fixieren. Der Kirschner-Draht kann beispielsweise einen Durchmesser von 0.5 mm bis 2 mm, insbesondere im Wesentlichen 1 mm, aufweisen.

Die vorliegende Erfindung wird anhand von in den Zeichnungen dargestellten Ausführungsbeispielen weiter erläutert, auf die sie jedoch nicht beschränkt sein soll.
Fig. 1 zeigt eine schematische Darstellung eines Darmbeins.
Fig. 2 zeigt ein Darmbein mit einer implantierten Osteosynthese-Platte nach einer Pelvic-Osteotomie.
Fig. 3 zeigt eine Schablone, die am Darmbein angelegt ist.
Fig. 4 zeigt eine Schablone mit einem Positioniergriff und einem Ausrichtstab am Darmbein
Fig. 5 zeigt eine andere Perspektive der Schablone aus Fig. 4.
Fig. 6 zeigt eine weitere Perspektive der Schablone aus Fig. 4 und Fig. 5.
Fig. 7 zeigt eine an einem Darmbein fixierte Schablone mit einem Meißel zum Markieren einer Schnittlinie.
Fig. 8 zeigt ein Darmbein mit zwei Bohrungen.

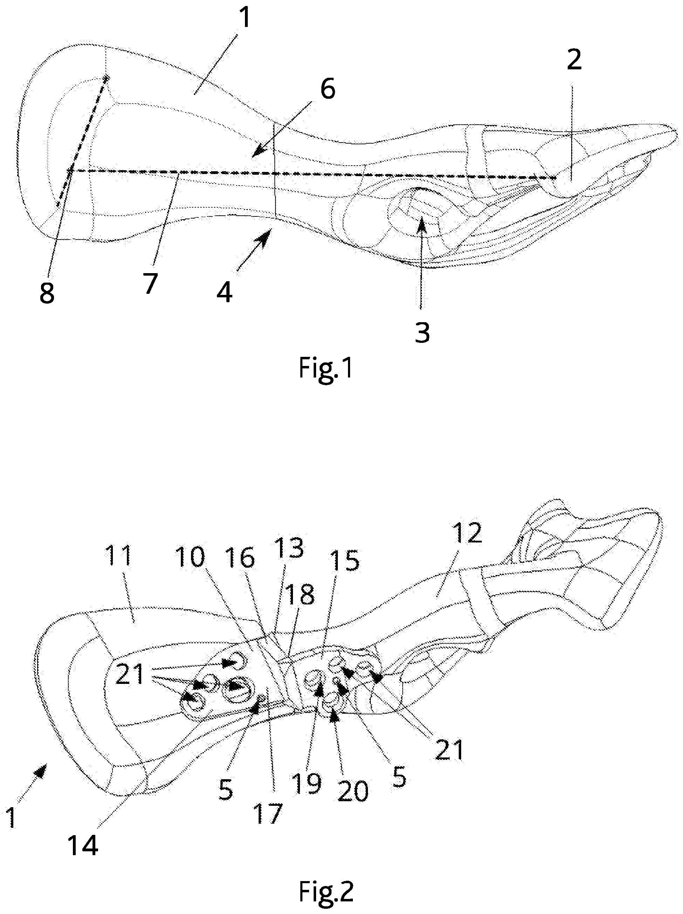

In Fig. 1 ist schematisch ein Darmbein 1 dargestellt, an dem, beispielsweise im Rahmen einer MPO, DPO oder TPO, eine Pelvic-Osteotomie durchgeführt werden kann. Das Darmbein 1 weist unter anderem einen Sitzbeinhöcker 2, ein Acetabulum 3 und eine ventrale Darmbeinkante 4 auf. Das Darmbein 1 weist des Weiteren einen lateralen Bereich 6 auf, der im Wesentlichen plan ist. Die Pelvic-Osteotomie kann normal zu einer imaginären Linie 7 erfolgen, welche cranial vom Übergang des mittleren in das ventrale Drittel der Darmbeinschaufel 8 und caudal auf Höhe des Sitzbeinhöckers 2 verläuft. Als weitere Orientierungshilfe verläuft die Pelvic-Osteotomie in der Regel auf Höhe oder knapp caudal des Verlaufes des N. gluteus cranialis.

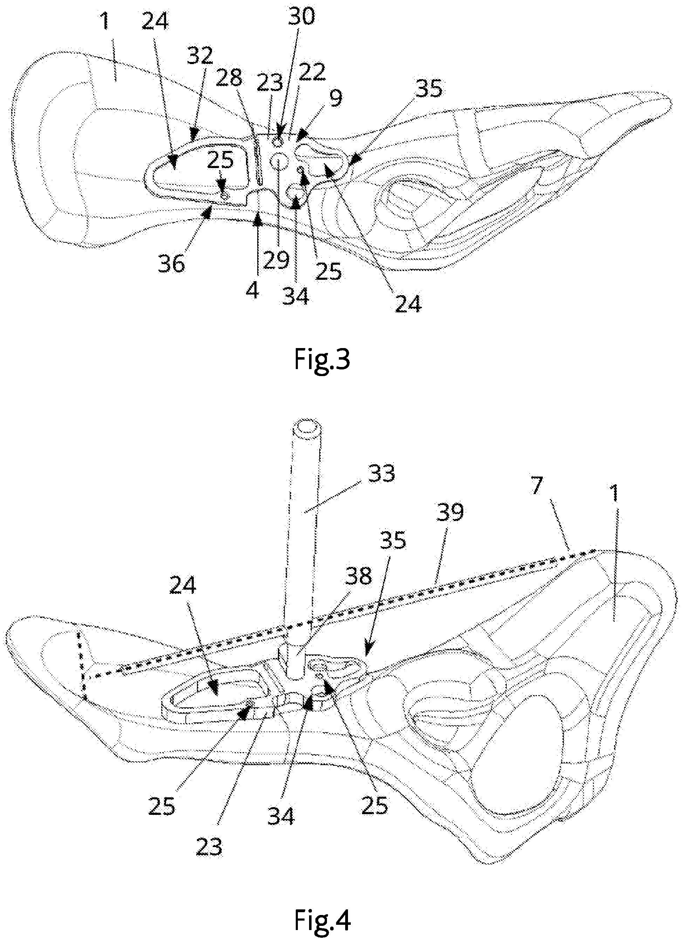

Zur Durchführung einer Pelvic-Osteotomie am Darmbein 1 erfolgt ein lateraler Zugang zum Darmbein 1. Es erfolgt eine Hautinzision zwischen der Spina iliaca cranialis ventralis und dem Trochanter major. Nach Durchtrennung der Haut und Subkutis wird die Fascia glutea und anschließend zwischen dem M. gluteus medius und M. tensor fascia latae präpariert. Es erfolgt eine Inzision an der gut tastbaren ventralen Darmbeinkante 4 des Darmbeins 1 caudal der Vv. gluteae craniales bzw. des N. gluteus, welche während des gesamten Eingriffes geschont werden. Der M. gluteus profundus wird mit einem Raspatorium von ventral nach dorsal vom Ileum abgelöst und gemeinsam mit dem M. gluteus medius nach dorsal verlagert (engl. "gluteal roll-up"). Bei Verwendung der Schablone 9 (siehe Figur 3) kann der Zugang so erfolgen, dass der Ursprung des M. gluteus medius im cranialen Bereich des Darmbeins 1 nicht oder kaum mobilisiert werden muss, in vielen Fällen ist auch eine Durchtrennung der Vv. ileolumbales nicht erforderlich. Durch Abduktion und Beugung der Extremität kann der Zug auf die Mm. glutei und den N. gluteus cranialis vermindert werden. Es kann ein modifizierter Hohmann Retraktor von medial caudal des N. gluteus cranialis an der Incisura ischiadica major platziert werden und zur Retraktion der Mm. glutei und damit zur Darstellung des lateralen Bereiches 6 des Darmbeins 1 dienen.

Fig. 2 zeigt das Darmbein 1 aus Fig. 1 nachdem die Pelvic-Osteotomie durchgeführt wurde und eine Osteosynthese-Platte 10 angelegt wurde. Dazu wurde am Darmbein 1 ein Schnitt gesetzt, wodurch das Darmbein 1 in ein craniales unteres Knochenfragment 11 und ein caudales oberes Knochenfragment 12 geteilt ist. Das obere Knochenfragment 11 und das untere Knochenfragment 12 werden im Rahmen der Operation zueinander versetzt angeordnet. Das untere Knochenfragment 11 und das obere Knochenfragment 12 weisen eine Schnittfläche 13 auf. Die Osteosynthese-Platte 10 weist einen im Wesentlichen planen unteren Plattenabschnitte 14 und einen im Wesentlichen planen oberen Plattenabschnitte 15 und einem Verbindungsabschnitt 16 auf. Die Osteosynthese-Platte 10 weist eine Oberseite 17 und eine gegenüberliegende Unterseite (nicht gezeigt) auf. Die Unterseite der Osteosynthese-Platte 10 liegt im bestimmungsgemäßen Gebrauch wie in Fig. 2 gezeigt am Darmbein 1 an. Dabei liegt die Unterseite des Verbindungsabschnitts 16 an der Schnittfläche 13 des oberen Knochenfragments 12 an. Am oberen Knochenfragment 12 liegt weiters die Unterseite des oberen Plattenabschnitts 15 an. Dementsprechend liegt die Unterseite des unteren Plattenabschnitts 14 am unteren Knochenfragment 11 an. Am Übergang des oberen Plattenabschnitts 15 zum Verbindungsabschnitt 16 weist die Unterseite der Osteosynthese-Platte 10 eine Innenkante auf, deren Ende 18 zu sehen ist. Die Innenkante schließt im Wesentlichen formschlüssig mit dem oberen Knochenfragment 12 ab. Daher verläuft der Schnitt durch das Darmbein 1 an der Stelle, an der später die Innenkante der Osteosynthese-Platte 10 angeordnet sein soll. Die Innenkante ist daher gleichzeitig die Versetzungslinie. Die Osteosynthese-Platte 10 weist weiters eine erste Durchgangsbohrung 19, eine zweite Durchgangsbohrung 20 und weitere Durchgangsbohrungen 21 auf, mittels derer die Osteosynthese-Platte 10 beispielsweise mit Schrauben am Darmbein 1 fixiert werden kann. Die Durchmesser der ersten Durchgangsbohrung 19, der zweiten Durchgangsbohrung 20 und der weiteren Durchgangsbohrungen 21 können variieren bzw. voneinander abweichen. Die erste Durchgangsbohrung 19 ist bevorzugt die Durchgangsbohrung mit dem niedrigsten Normalabstand zur Innenkante auf dem oberen Plattenabschnitt 15. Weiters sind zwei Fixationslöcher 5 vorgesehen, durch die jeweils ein Fixiermittel, wie beispielsweise ein Kirschner-Draht, eine Fixierung der Osteosynthese-Platte 10 am Darmbein 1 ermöglichen kann.

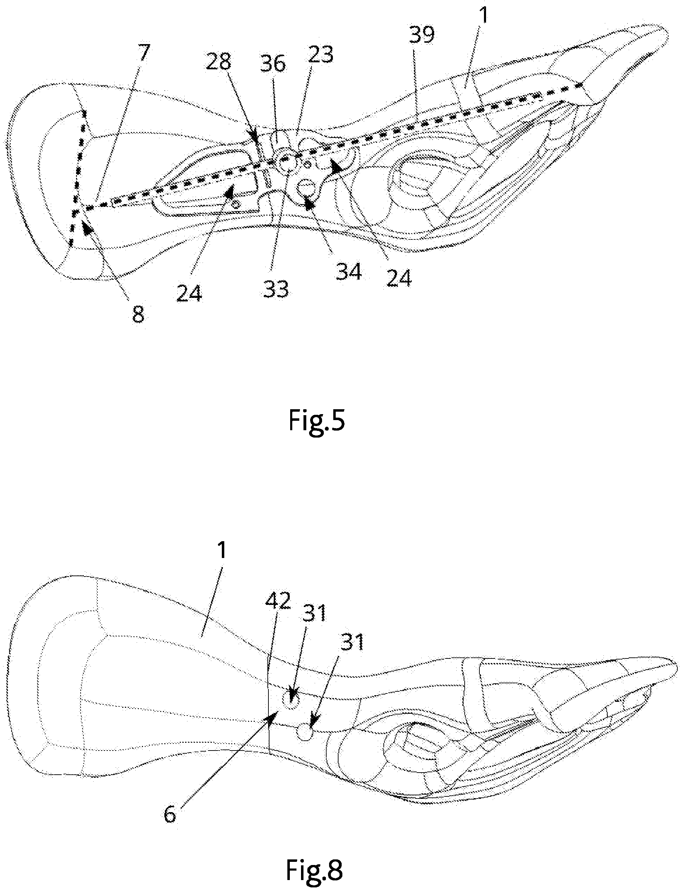

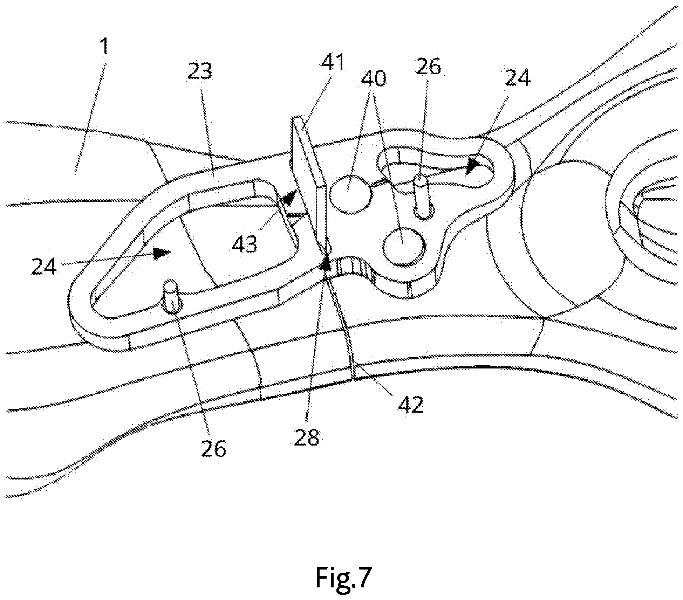

Fig. 3 zeigt eine an die Osteosynthese-Platte 10 angepasste Schablone 9 mit einer Schablonenplatte 23, die am lateralen Bereich 6 des Darmbeins 1 angelegt ist. Die Schablonenplatte 23 weist zwei Aussparungen 24 auf, die eine Palpation des Darmbeins 1 erlauben und/oder als Sichtfenster fungieren, wenn die Schablonenplatte 23 am Darmbein 1 angelegt ist. Weiters sind zwei Löcher 25 vorgesehen, durch die jeweils ein Fixiermittel 26 (siehe Fig. 7), wie beispielsweise ein Kirschner-Draht, eine Fixierung der Schablonenplatte 23 am Darmbein 1 ermöglichen kann. Die Schablonenplatte 23 hat eine Unterseite (nicht gezeigt) und eine Oberseite 22 und weist weiters ein Langloch 28 auf, wobei das Langloch 28 von der Oberseite 22 zu der Unterseite der Schablonenplatte 23 reicht und das Langloch 28 das Markieren einer Schnittlinie 42 (siehe Fig. 8) am Darmbein 1 durch das Langloch 28 hindurch ermöglicht. Die Oberseite 22 liegt im Verhältnis zur Unterseite der Schablonenplatte 23 lateral. Die Schablonenplatte 23 weist weiters ein erstes Rundloch 29 auf, wobei das erste Rundloch 29 von der Oberseite 22 der Schablonenplatte 23 zu einer Unterseite (nicht gezeigt) der Schablonenplatte 23 reicht und durch das erste Rundloch 29 eine Bohrung 31 (siehe Fig.8) am Darmbein 1 gesetzt werden kann. Die Position und Ausrichtung des Langlochs 28 und des ersten Rundlochs 29 ist durch eine Projektion der Osteosynthese-Platte 10 auf die Schablonenplatte 23 bestimmt. Die Position des ersten Rundlochs 29 entspricht der Position eines durch die Projektion erzeugten Bildes der ersten Durchgangsbohrung 19 auf der Schablonenplatte 23. Die Position und Ausrichtung des Langlochs 28 entspricht der Position und Ausrichtung des Bildes der Versetzungslinie bzw. der Innenkante auf der Schablonenplatte 23. Die Projektion ist eine Orthogonalprojektion, wobei für die Projektion die Haupterstreckungsebene des oberen Plattenabschnitts 15 der Osteosynthese-Platte parallel zur Haupterstreckungsebene der Schablonenplatte 23 ist. Ein Umriss 32 der Schablonenplatte 23 ist derart, dass der Umriss 32 im Wesentlichen einer Außenkontur des durch die Projektion erzeugten Bildes der Osteosynthese-Platte 10 auf der Schablonenplatte 23 entspricht. Durch das erste Rundloch 29 kann ein Positioniergriff 33 (siehe Fig. 4) eingeführt bzw. mit der Schablonenplatte 23 lösbar verbunden werden. Der Positioniergriff 33 kann die Positionierung der Schablonenplatte 23 am Darmbein 1 vereinfachen. Die Schablonenplatte 23 weist ein weiteres Loch 30 auf.

Die Schablonenplatte 23 weist ein zweites Rundloch 34 auf, wobei die Position des zweiten Rundlochs 34 der Position eines durch die Projektion erzeugten Bildes einer zweiten Durchgangsbohrung 20 auf der Schablonenplatte 23 entspricht. Durch das zweite Rundloch 34 kann ebenso wie durch das erste Rundloch 29 eine Bohrung 31 (siehe Fig. 8) am bzw. im Darmbein 1 gesetzt werden.

Die Schablonenplatte 23 ist mittels subtraktiver Methoden, in diesem Fall Fräsen und Bohren, aus einer Platte aus Titan gefertigt. Die Schablonenplatte 23 besteht folglich aus Titan und hat eine Höhe bzw. Dicke von 3 mm und bietet daher in bildgebenden Verfahren auf Basis von Röntgenstrahlung, beispielsweise mittels C-Bogen, einen ausreichenden Kontrast zum Darmbein 1 und zum Umfeld des Darmbeins 1, um die Positionierung der Schablonenplatte 23 am Darmbein 1 kontrollieren zu können. Mittels intraoperativer Durchleuchtung, beispielsweise mittels C-Bogen, kann die Position der röntgendichten Schablone 9 überprüft werden. Die Schablonenplatte 23 ist im Wesentlichen röntgendicht bzw. ausreichend röntgendicht in diesem Zusammenhang. Die Osteosynthese-Platte 10 und die Schablone 9 bilden gemeinsam ein Kit (nicht gezeigt) für eine Pelvic-Osteotomie.

Die Schablone 9 liegt cranial des Ursprunges des M. rectus femoris lateral am Darmbein 1 und kann wie folgt eingerichtet werden: Ein caudales Ende 35 der Schablonenplatte 23 befindet sich am cranialen Ursprung des M. rectus femoris. Die ventrale Kontur 36 der Schablonenplatte 23 schließt mit oder knapp dorsal der ventralen Darmbeinkante 4 ab. Das caudale Ende 35 der Schablonenplatte 23 ist leicht abgeschrägt, um besser am Darmbein 1 anzuliegen. Das caudale Ende 35 kann alternativ nach oben gebogen sein.

Fig. 4 zeigt im Vergleich zu Fig. 3, einen an der Schablone 9 angeordneten Positioniergriff 33, mit dessen Hilfe die Ausrichtung der Schablonenplatte 23 in Relation zum Darmbein 1 vereinfacht werden kann. Der Positioniergriff 33 weist einen zylinderförmigen Abschnitt 38 auf, der in das erste Rundloch 29 eingeführt ist und im Wesentlichen formschlüssig in das erste Rundloch 29 eingreift, wodurch der Positioniergriff lösbar mit der Schablonenplatte 23 verbunden ist. Der Positioniergriff 33 ist abnehmbar. Nach der Positionierung der Schablonenplatte 23 und beispielsweise einer Fixierung mit einem Fixiermittel 26, wie beispielsweise mit Kirschner-Drähten, (siehe Fig. 7) kann der Positioniergriff 33 abgenommen werden und es kann eine Bohrung 31 durch das erste Rundloch 29 und das zweite Rundloch 34 am Darmbein 1 gesetzt werden.

Der Positioniergriff 33 weist des Weiteren einen Ausrichtstab 39 auf. Wie in Fig. 2 zu sehen ist, kann die Pelvic-Osteotomie normal zu einer imaginären Linie 7 erfolgen, welche cranial vom Übergang des mittleren in das ventrale Drittel der Darmbeinschaufel 8 und caudal auf Höhe des Sitzbeinhöckers 2 verläuft. Der Ausrichtstab 39 kann während einer Operation entlang dieser imaginären Linie 7 ausgerichtet werden. Als weitere Orientierungshilfe verläuft die Pelvic-Osteotomie in der Regel auf Höhe oder knapp caudal des Verlaufes des N. gluteus cranialis. Der Ausrichtstab 39 ist abnehmbar und kann beispielsweise durch ein Loch (nicht gezeigt) im Positioniergriff 33 in den Positioniergriff 33 eingebracht bzw. entnommen werden. Die Hauptachse des Ausrichtstabs 39 steht normal auf die Hauptachse des Positioniergriff 33. Der Positioniergriff 33 weist einen vom zylindrischen Abschnitt 38 beanstandeten Stift (nicht gezeigt) auf, der in das weitere Loch 30 der Schablonenplatte 23 eingreifen kann und in der Darstellung in Fig. 3 eingreift. Die Hauptachse des Stifts verläuft parallel zur Hauptachse des zylindrischen Abschnitts 28. Der Abstand des weiteren Lochs 30 zum ersten Rundloch 29 entspricht dem Abstand des zylindrischen Abschnitts 38 zum Stift, um ein gleichzeitiges Eingreifen des Stifts in das weitere Loch 30 und des zylindrischen Abschnitts 38 in das erste Rundloch 29 zu ermöglichen. Der Stift ist mittels eines Verbindungsstücks 37 mit dem zylindrischen Abschnitt 38 verbunden. Durch den Stift, der in das weitere Loch 30 eingreift, ist der Positioniergriff 33 nicht um eine Hauptachse des ersten Rundlochs 29 drehbar. Dadurch kann eine geometrische Beziehung zwischen der Ausrichtung des Ausrichtstabs 39 und der Ausrichtung der Schablonenplatte 23 festgelegt werden und insgesamt die korrekte Positionierung der Schablonenplatte vereinfacht werden.

Fig. 5 zeigt die eine Draufsicht auf die Schablone 9 aus Fig. 4. Der Ausrichtstab 39 ist entlang der imaginären Linie 7 ausgerichtet Das Langloch 28 verläuft im Wesentlichen normal auf die imaginäre Linie 7 und dadurch auch auf den Ausrichtstab 39. Dadurch bedingt steht auch ein Schnitt entlang einer mittels des Langlochs 28 markierten Schnittlinie 42 (siehe Figur 8) im Wesentlichen normal auf die imaginäre Linie 7. Der Winkel zwischen der imaginären Linie 7 und der Schnittline 42 bzw. der Schnittfläche 13 kann beispielsweise 85° bis 95° betragen.

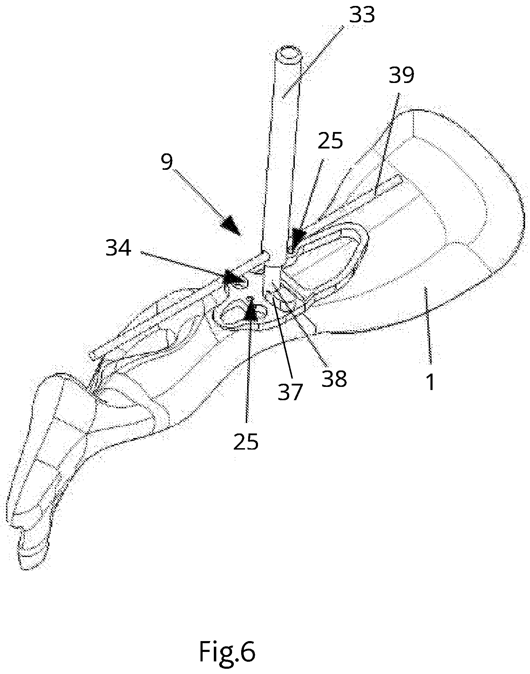

Fig. 6 zeigt eine weitere Perspektive der Schablone 9 aus den Figuren 4 und 5. Der Ausrichtstab 39 vereinfacht die Ausrichtung der Schablone 9.

Fig. 7 zeigt eine Schablonenplatte 23, die mittels zweier Fixiermittel 26, in diesem Fall Kirschner-Drähte, durch zwei Löcher 25 am Darmbein 1 fixiert ist. Durch das erste Rundloch 29 und ein zweites Rundloch 34 wurden zwei Bohrungen 31 mittels winkelstabiler Bohrhülsen gesetzt. Eine der Bohrungen 31 kann cis- und eine Bohrung 31 kann trans-kortikal gebohrt sein. Zur zwischenzeitlichen Fixation der Schablonenplatte 23 und Vorbohrung in der korrekten Position wurde je eine entsprechende Schraube 40 in die Bohrungen 31 bzw. das erste Rundloch 29 und das zweite Rundloch 34 eingebracht. Die Aussparungen 24 erlauben eine Palpation des Darmbeins 1 und eine verbesserte Sicht. Mittels eines Meißels 41 kann durch das Langloch 28 eine Schnittlinie 42 (siehe Figur 8) am Darmbein markiert werden. Die Breite des Meißels 41 entspricht im Wesentlichen der Breite des Langlochs 28. Dadurch fungiert die Schablonenplatte 23 mit dem Langloch 28 auch als Meißelführung 43 für den Meißel 41. Das Markieren der Schnittlinie 42 kann durch Anritzen des Darmbeins 1 mittels des Meißels 41 vorgenommen werden. Alternativ kann auch bereits zumindest ein Teil des Schnitts durch das Langloch 28 bzw. die Meißelführung 43 hindurch gesetzt werden.

Fig. 8 zeigt ein Darmbein 1 mit zwei Bohrungen 31, die mittels der Schablone 9 gesetzt wurden. Die Schnittlinie 42 wurde markiert. Nach dem Durchtrennen des Darmbeins 1 entlang der Schnittlinie 42, kann die Osteosynthese-Platte 10 aus Fig. 2 implantiert werden. Die Pelvic-Osteotomie erfolgt mit Meißeln 41 entlang der markierten Schnittlinie 42 normal zum Darmbein 1. Das Darmbein 1 kann auch als Os Ileum bezeichnet werden. Zur Verhinderung einer nach der Rotation entstehenden und die Glutealmuskulatur irritierenden Knochenkante bzw. Knochenspitze kann der Schnitt im letzten Viertel des dorsalen Bereiches im Winkel von etwa 45° nach caudal abweichen. Durch die Verwendung von scharfen und dünnen, beispielsweise 0,5 mm bis 1 mm breiten, Meißeln 41 ist eine sehr exakte Osteotomie möglich. Die Hitzeentwicklung ist im Vergleich zum Sägen vernachlässigbar.

Nachdem der Schnitt vollständig gesetzt wurde, kann das caudale obere Knochenfragment 12 mittels einer Zweipunktzange und/oder eines von cranial eingebrachten Meißels 41 angehoben bzw. nach lateral verlagert werden und der caudale obere Plattenabschnitt 15 der Osteosynthese-Platte 10 temporär mit zwei Kortikalisschrauben oder Schrauben mit sphärischen Beilagscheiben am Darmbein 1 fixiert werden. Die verbleibenden bzw. noch zu setzenden Bohrungen 31 am oberen Knochenfragment 12 werden durch winkelstabile Bohrhülsen durch die weiteren 21 Durchgangsbohrungen des oberen Plattenabschnitts 15 der Osteosynthese-Platte 10 gebohrt und alle Bohrungen 31 mit winkelstabilen bikortikalen Schrauben besetzt. Damit ist die Osteosynthese-Platte 10 fest am caudalen oberen Knochenfragment 12 fixiert. Mittels eines Drehhebels, welcher winkelstabil in einer Bohrung 31 fixiert werden kann, kann das caudale obere Knochenfragment 12 nach ventral rotiert werden. Beispielsweise durch eine Kortikalisschraube kann die Osteosynthese-Platte 10 im Bereich des cranialen unteren Knochenfragments 12 direkt an das Darmbein 1 gepresst werden. Dann werden die verbleibenden cranialen Bohrungen 31 mit winkelstabilen Schrauben im Darmbein 1 besetzt. Es ist darauf zu achten, dass die cranialen Schrauben nicht das Kreuzdarmbeingelenk invadieren.

Die Osteosynthese-Platte 10 kann in diesem Beispiel mittels zwei Schrauben (nicht gezeigt), die in die erste Durchgangsbohrung 19 und die zweite Durchgangsbohrung 20 sowie in jeweils eine der Bohrungen 31 eingreifen, am Darmbein 1 befestigt werden. Weitere Bohrungen 31 können durch weitere Durchgangsbohrungen 21 gesetzt werden. Die Anzahl an Rundlöchern in der Schablonenplatte 23 kann kleiner sein als die Summe der Anzahl der ersten Durchgangsbohrung 19, der zweiten Durchgangsbohrung 20 und der weiteren Durchgangsbohrungen 21 in der Osteosynthese-Platte 10.

Die Schablone 9 bzw. die Schablonenplatte 23 ist an die Osteosynthese-Platte 10 angepasst. Im Vergleich zur Osteosynthese-Platte 10 kann die Schablone 9 jedoch wiederverwendet werden und im Rahmen mehrerer Implantationen von baugleichen Osteosynthese-Platten 10 verwendet werden. Entscheidend für die Funktionsweise der Schablone 9 ist, dass die Position des Langlochs 28 relativ zur Position des zumindest einen ersten Rundlochs 29 an die Osteosynthese-Platte 10 angepasst ist, um die Implantation der Osteosynthese-Platte 10 zu vereinfachen. Daher kann eine Schablone 9 in Kombination mit mehreren verschiedene Osteosynthese-Platten 10 verwendet werden, deren Verhältnis der Position und Ausrichtung der Versetzungslinie und zumindest der ersten Durchgangsbohrung 19 ident sind. Beispielsweise kann der Normalabstand der Versetzungslinie zum Mittelpunkt der ersten Durchgangsbohrung 19 ident sein. Es gibt also verschieden geformte Osteosynthese-Platten 10, die unter Zuhilfenahme derselben Schablone 9 verwendet werden können.

## Patentansprüche

1. Schablone (9) zur Markierung einer Schnittlinie (42) und zum Setzen von zumindest einer Bohrung (31) am Darmbein (1) zur Implantation einer Osteosynthese-Platte (10) bei einer Pelvic-Osteotomie,
wobei die Schablone (9) eine Schablonenplatte (23) mit einer Oberseite (22) und einer Unterseite aufweist, wobei die Schablonenplatte (23) ein Langloch (28) aufweist, wobei das Langloch (28) von der Oberseite (22) zu der Unterseite der Schablonenplatte (23) reicht und das Langloch (28) das Markieren einer Schnittlinie (42) am Darmbein (1) durch das Langloch (28) hindurch ermöglicht, und wobei die Schablonenplatte (23) zumindest ein erstes Rundloch (29) aufweist, wobei das zumindest eine erste Rundloch (29) von der Oberseite (22) der Schablonenplatte (23) zu der Unterseite der Schablonenplatte (23) reicht und durch das erste Rundloch (29) eine Bohrung (31) am Darmbein (1) gesetzt werden kann, **dadurch gekennzeichnet, dass** die Schablone (9) an der Oberseite (22) der Schablonenplatte (23) einen abnehmbaren Positioniergriff (33) aufweist, der zur Positionierung der Schablonenplatte (23) am Darmbein (1) verwendbar ist, und dass der Positioniergriff (33) einen Ausrichtstab (39) aufweist, wobei die Hauptachse des Ausrichtstabs (39) normal auf die Hauptachse des Positioniergriffs (33) steht.

2. Kit für eine Pelvic-Osteotomie, umfassend:
- eine Osteosynthese-Platte (10) zur Implantation an einem Darmbein (1) bei einer Pelvic-Osteotomie mit zwei im Wesentlichen planen Plattenabschnitten (14, 15), welche entlang einer Versetzungslinie zueinander versetzt sind, und mit mindestens einer ersten Durchgangsbohrung (19) zur Befestigung eines Knochenfragments (11, 12), beispielsweise mittels zumindest einer Schraube;
- eine an die Osteosynthese-Platte (10) angepasste Schablone (9), wobei die Schablone (9) eine Schablonenplatte (23) mit einer Oberseite (22) und einer Unterseite aufweist, wobei die Schablonenplatte (23) ein Langloch (28) aufweist, wobei das Langloch (28) von der Oberseite (22) zu der Unterseite der Schablonenplatte (23) reicht und das Langloch (28) das Markieren einer Schnittlinie (42) am Darmbein (1) durch das Langloch (28) hindurch ermöglicht, und wobei die Schablonenplatte (23) zumindest ein erstes Rundloch (29) aufweist, wobei das zumindest eine erste Rundloch (29) von der Oberseite (22) der Schablonenplatte (23) zu der Unterseite der Schablonenplatte (23) reicht und durch das erste Rundloch (29) eine Bohrung (31) am Darmbein (1) gesetzt werden kann,
wobei die Position und Ausrichtung des Langlochs (28) und des zumindest einen ersten Rundlochs (29) durch eine Projektion der Osteosynthese-Platte (10) auf die Schablonenplatte (23) bestimmt ist, wobei die Position des ersten Rundlochs (29) der Position eines durch die Projektion erzeugten Bildes der ersten Durchgangsbohrung (19) auf der Schablonenplatte (10) entspricht und wobei die Position und Ausrichtung des Langlochs (28) der Position und Ausrichtung des Bildes der Versetzungslinie auf der Schablonenplatte (23) entspricht, wobei die Schablone (9) an der Oberseite (22) der Schablonenplatte (23) einen abnehmbaren Positioniergriff (33) aufweist, der zur Positionierung der Schablonenplatte (23) am Darmbein (1) verwendbar ist, **dadurch gekennzeichnet, dass** der Positioniergriff (33) einen Ausrichtstab (39) aufweist, wobei die Hauptachse des Ausrichtstabs (39) normal auf die Hauptachse des Positioniergriffs (33) steht.

3. Kit nach Anspruch 2, **dadurch gekennzeichnet, dass** die Osteosynthese-Platte (10) mindestens eine weitere Durchgangsbohrung (21) zur Befestigung eines Knochenfragments (11, 12) aufweist und die Schablonenplatte (23) zumindest ein weiteres Rundloch aufweist, wobei das weitere Rundloch im Vergleich zum ersten Rundloch (29) an einer gegenüberliegenden Seite der Schablonenplatte (23) bezüglich des Langloches (28) angeordnet ist, wobei die Position des weiteren Rundlochs (28) der Position eines durch die Projektion erzeugten Bildes der weiteren Durchgangsbohrung (21) auf der Schablonenplatte (23) entspricht.

4. Kit nach einem der Ansprüche 2 oder 3 oder Schablone (9) nach Anspruch 1, **dadurch gekennzeichnet, dass** die Schablonenplatte (23) aus röntgendichtem Material besteht, beispielsweise aus Stahl.

5. Kit nach einem der Ansprüche 2 bis 4 oder Schablone (9) nach Anspruch 1, **dadurch gekennzeichnet, dass** an der Oberseite (22) der Schablonenplatte (23) zumindest eine Bohrführung angeordnet ist, wobei die Hauptachse der zumindest einen Bohrführung mit der Hauptachse des zumindest einen ersten Rundlochs (29) zusammenfällt.

6. Kit nach einem der Ansprüche 2 bis 5 oder Schablone (9) nach Anspruch 1, **dadurch gekennzeichnet, dass** die Schablonenplatte (23) zumindest eine Aussparung (24) aufweist, die eine Palpation des Darmbeins (1) erlaubt, wenn die Schablonenplatte (23) am Darmbein (1) angelegt ist.

7. Kit nach einem der Ansprüche 2 bis 6 oder Schablone (9) nach Anspruch 1, **dadurch gekennzeichnet, dass** die Schablonenplatte (23) zumindest zwei Löcher (25) aufweist, durch die jeweils ein Fixiermittel (26), vorzugsweise ein Kirschner-Draht, eine Fixierung der Schablonenplatte (23) am Darmbein (1) ermöglichen kann.

## Claims

1. A template (9) for marking a cutting line (42) and for placing at least one bore (31) on the ilium (1) for the implantation of an osteosynthesis plate (10) in a pelvic osteotomy, wherein the template (9) comprises a template plate (23) having an upper side (22) and a lower side, wherein the template plate (23) comprises an elongated hole (28), wherein the elongated hole (28) extends from the upper side (22) to the lower side of the template plate (23) and the elongated hole (28) enables the marking of a cutting line (42) on the ilium (1) through the elongated hole (28), and wherein the template plate (23) comprises at least one first round hole (29), wherein the at least one first round hole (29) extends from the upper side (22) of the template plate (23) to the lower side of the template plate (23) and a bore (31) can be placed on the ilium (1) through the first round hole (29), wherein the template (9) comprises on the upper side (22) of the template plate (23) a detachable positioning handle (33), which is usable for positioning the template plate (23) on the ilium (1),
**characterized in that** the positioning handle (33) comprises an alignment rod (39), wherein the main axis of the alignment rod (39) is perpendicular to the main axis of the positioning handle (33).

2. A kit for a pelvic osteotomy, comprising:
- an osteosynthesis plate (10) for implantation on an ilium (1) in a pelvic osteotomy, having two substantially planar plate sections (14, 15) which are offset relative to each other along an offset line, and having at least one first through-bore (19) for attaching a bone fragment (11, 12), for example by means of at least one screw;
- a template (9) adapted to the osteosynthesis plate (10),
wherein the template (9) comprises a template plate (23) having an upper side (22) and a lower side, wherein the template plate (23) comprises an elongated hole (28), wherein the elongated hole (28) extends from the upper side (22) to the lower side of the template plate (23) and the elongated hole (28) enables the marking of a cutting line (42) on the ilium (1) through the elongated hole (28), and wherein the template plate (23) comprises at least one first round hole (29), wherein the at least one first round hole (29) extends from the upper side (22) of the template plate (23) to the lower side of the template plate (23) and a bore (31) can be placed on the ilium (1) through the first round hole (29),
wherein the position and alignment of the elongated hole (28) and of the at least one first round hole (29) is determined by a projection of the osteosynthesis plate (10) onto the template plate (23), wherein the position of the first round hole (29) corresponds to the position of an image of the first through-bore (19) generated by the projection on the template plate (10) and wherein the position and alignment of the elongated hole (28) corresponds to the position and alignment of the image of the offset line on the template plate (23), wherein the template (9) comprises on the upper side (22) of the template plate (23) a detachable positioning handle (33), which is usable for positioning the template plate (23) on the ilium (1),
**characterized in that** the positioning handle (33) comprises an alignment rod (39), wherein the main axis of the alignment rod (39) is perpendicular to the main axis of the positioning handle (33).

3. The kit according to claim 2, **characterized in that** the osteosynthesis plate (10) comprises at least one further through-bore (21) for attaching a bone fragment (11, 12) and the template plate (23) comprises at least one further round hole, wherein the further round hole, in comparison to the first round hole (29), is arranged on an opposite side of the template plate (23) with respect to the elongated hole (28), wherein the position of the further round hole (28) corresponds to the position of an image of the further through-bore (21) generated by the projection on the template plate (23).

4. The kit according to any one of claims 2 or 3 or the template (9) according to claim 1, **characterized in that** the template plate (23) consists of radiopaque material, for example of steel.

5. The kit according to any one of claims 2 to 4 or the template (9) according to claim 1, **characterized in that** at least one drill guide is arranged on the upper side (22) of the template plate (23), wherein the main axis of the at least one drill guide coincides with the main axis of the at least one first round hole (29).

6. The kit according to any one of claims 2 to 5 or the template (9) according to claim 1, **characterized in that** the template plate (23) comprises at least one recess (24) which allows a palpation of the ilium (1) when the template plate (23) is applied to the ilium (1).

7. The kit according to any one of claims 2 to 6 or the template (9) according to claim 1, **characterized in that** the template plate (23) comprises at least two holes (25) through which in each case a fixation means (26), preferably a Kirschner wire, can enable a fixation of the template plate (23) to the ilium (1).

## Revendications

1. Gabarit (9) pour le marquage d'une ligne de coupe (42) et pour la réalisation d'au moins un perçage (31) sur l'ilium (1) pour l'implantation d'une plaque d'ostéosynthèse (10) lors d'une ostéotomie pelvienne,
dans lequel le gabarit (9) comprend une plaque de gabarit (23) avec une face supérieure (22) et une face inférieure, dans lequel la plaque de gabarit (23) comprend un trou oblong (28), dans lequel le trou oblong (28) s'étend de la face supérieure (22) à la face inférieure de la plaque de gabarit (23) et le trou oblong (28) permet le marquage d'une ligne de coupe (42) sur l'ilium (1) à travers le trou oblong (28), et dans lequel la plaque de gabarit (23) comprend au moins un premier trou rond (29), dans lequel l'au moins un premier trou rond (29) s'étend de la face supérieure (22) de la plaque de gabarit (23) à la face inférieure de la plaque de gabarit (23) et un perçage (31) peut être réalisé sur l'ilium (1) à travers le premier trou rond (29), dans lequel le gabarit (9) comprend, sur la face supérieure (22) de la plaque de gabarit (23), une poignée de positionnement (33) amovible qui est utilisable pour le positionnement de la plaque de gabarit (23) sur l'ilium (1),
**caractérisé en ce que** la poignée de positionnement (33) comprend une tige d'alignement (39), dans lequel l'axe principal de la tige d'alignement (39) est perpendiculaire à l'axe principal de la poignée de positionnement (33).

2. Kit pour une ostéotomie pelvienne, comprenant :
- une plaque d'ostéosynthèse (10) pour l'implantation sur un ilium (1) lors d'une ostéotomie pelvienne avec deux sections de plaque (14, 15) sensiblement planes, lesquelles sont décalées l'une par rapport à l'autre le long d'une ligne de décalage, et avec au moins un premier perçage traversant (19) pour la fixation d'un fragment osseux (11, 12), par exemple au moyen d'au moins une vis ;
- un gabarit (9) adapté à la plaque d'ostéosynthèse (10), dans lequel le gabarit (9) comprend une plaque de gabarit (23) avec une face supérieure (22) et une face inférieure, dans lequel la plaque de gabarit (23) comprend un trou oblong (28), dans lequel le trou oblong (28) s'étend de la face supérieure (22) à la face inférieure de la plaque de gabarit (23) et le trou oblong (28) permet le marquage d'une ligne de coupe (42) sur l'ilium (1) à travers le trou oblong (28), et dans lequel la plaque de gabarit (23) comprend au moins un premier trou rond (29), dans lequel l'au moins un premier trou rond (29) s'étend de la face supérieure (22) de la plaque de gabarit (23) à la face inférieure de la plaque de gabarit (23) et un perçage (31) peut être réalisé sur l'ilium (1) à travers le premier trou rond (29), dans lequel la position et l'orientation du trou oblong (28) et de l'au moins un premier trou rond (29) sont déterminées par une projection de la plaque d'ostéosynthèse (10) sur la plaque de gabarit (23), dans lequel la position du premier trou rond (29) correspond à la position d'une image, générée par la projection, du premier perçage traversant (19) sur la plaque de gabarit (10) et dans lequel la position et l'orientation du trou oblong (28) correspondent à la position et à l'orientation de l'image de la ligne de décalage sur la plaque de gabarit (23), dans lequel le gabarit (9) comprend, sur la face supérieure (22) de la plaque de gabarit (23), une poignée de positionnement (33) amovible qui est utilisable pour le positionnement de la plaque de gabarit (23) sur l'ilium (1),
**caractérisé en ce que** la poignée de positionnement (33) comprend une tige d'alignement (39), dans lequel l'axe principal de la tige d'alignement (39) est perpendiculaire à l'axe principal de la poignée de positionnement (33).

3. Kit selon la revendication 2, **caractérisé en ce que** la plaque d'ostéosynthèse (10) comprend au moins un autre perçage traversant (21) pour la fixation d'un fragment osseux (11, 12) et la plaque de gabarit (23) comprend au moins un autre trou rond, dans lequel l'autre trou rond, par comparaison au premier trou rond (29), est disposé sur un côté opposé de la plaque de gabarit (23) par rapport au trou oblong (28), dans lequel la position de l'autre trou rond (28) correspond à la position d'une image, générée par la projection, de l'autre perçage traversant (21) sur la plaque de gabarit (23).

4. Kit selon l'une des revendications 2 ou 3 ou gabarit (9) selon la revendication 1, **caractérisé en ce que** la plaque de gabarit (23) est constituée d'un matériau radio-opaque, par exemple d'acier.

5. Kit selon l'une des revendications 2 à 4 ou gabarit (9) selon la revendication 1, **caractérisé en ce qu'**au moins un guide de perçage est disposé sur la face supérieure (22) de la plaque de gabarit (23), dans lequel l'axe principal de l'au moins un guide de perçage coïncide avec l'axe principal de l'au moins un premier trou rond (29).

6. Kit selon l'une des revendications 2 à 5 ou gabarit (9) selon la revendication 1, **caractérisé en ce que** la plaque de gabarit (23) comprend au moins un évidement (24) qui permet une palpation de l'ilium (1) lorsque la plaque de gabarit (23) est appliquée sur l'ilium (1).

7. Kit selon l'une des revendications 2 à 6 ou gabarit (9) selon la revendication 1, **caractérisé en ce que** la plaque de gabarit (23) comprend au moins deux trous (25) à travers chacun desquels un moyen de fixation (26), de préférence une broche de Kirschner, peut permettre une fixation de la plaque de gabarit (23) sur l'ilium (1).
